# EUROPEAN PATENT APPLICATION

(11) **EP 4 742 262 A1**
(43) Date of publication of application: **13.05.2026**
(21) Application number: 25210721.4
(22) Date of filing: 23.10.2025
(51) Int. Cl.: G16H 20/40

(54) **SAFETY MONITOR FOR HEART-LUNG MACHINE AND ASSOCIATED METHODS**

(30) Priority: 12.11.2024 US 202418944904
(71) Applicant: LivaNova Deutschland GmbH, 80939 Munich (DE)
(72) Inventor: Bauer, Hermann, 80639 München (DE); Schubert, Friedemann, 80639 München (DE)
(74) Representative: Grünecker Patent- und Rechtsanwälte PartG mbB

(57) **Abstract**

The present disclosure includes a system monitor and associated methods for a heart-lung machine (HLM) and/or other like device. Example system monitors may include a profile module adapted to accept user parameters and receive settings changes from a remote interface, and provide updated user parameters to a safety module based upon received settings changes. The system monitors of the present disclosure may include a subsystem module in communication with one or more subsystems. The subsystem module may be adapted to periodically scan subsystem data from the one or more subsystems and provide actual user parameters to the safety module from the scanned subsystem data. The safety module of the present disclosure may be configured to compare the updated user parameters to the actual user parameters, and correct the actual user parameters to match the updated user parameters.

## Description

### TECHNICAL FIELD

The present disclosure relates to the field of medical devices and associated medical systems. More particularly, the present disclosure relates to operative characteristics, designs, devices, methods of use, and methods of utilization for heart-lung machines, related components and other like devices.

### BACKGROUND

Heart-lung machine ("HLM") systems are used, along with an extracorporeal circuit and a hollow fiber oxygenator, to meet a patient's circulatory and blood gas exchange needs during medical procedures such as cardiopulmonary bypass ("CPB") surgery. Blood from the patient is either gravity drained, or VAVD (vacuum assisted venous drainage) is used, to obtain the desired amount of blood flow through the extracorporeal circuit. A pump, such as a peristaltic pump or a centrifugal pump, may be used in the extracorporeal circuit in order to pump blood from the patient, to the reservoir, through the oxygenator, and finally back to the patient. In addition to an HLM per se, heart-lung machine systems can include multiple types of patient monitoring devices that are used in conjunction with the HLM.

### BRIEF SUMMARY

During routine operation of a heart-lung machine or other like device, a user and/or practitioner inputs a user profile (i.e., profile) including user settings, and various operational settings related to the control assemblies of a heart-lung machine to ensure safe and effective operation of the heart-lung machine and all peripheral and like components related to the operation of the heart-lung machine and/or other like device(s). Certain heart-lung machines, however, are unable to automatically update operating parameters in response to a change in user and/or operational settings, and this can be problematic as it results in inaccurate, ineffective, and deleterious assessment of a user of a heart-lung machine and/or other like device which therefore may result in inaccurate, ineffective and deleterious treatment of the user. In other words, if accurate user settings and/or operational settings are not ensured automatically, and/or not immediately corrected by a human operator, the heart-lung machine and/or other like and/or connected components will not function properly, will not deliver accurate therapy to a user, and may result in dangerous and/or deleterious consequences for a user of a heart-lung machine and/or related device due to inaccuracy and/or faulty operation of the heart-lung machine and/or other like devices and/or other related components and/or devices.

The present disclosure provides a system monitor in addition to various other features that provide automatic correction of the user and/or operational settings of a heart-lung machine and/or other like device and/or other devices and/or components related to the use of a heart-lung machine and/or other like devices and/or systems.

An example system monitor of the present disclosure may include a profile module adapted and/or otherwise configured to accept user parameters and receive settings changes from a remote interface. The profile module of this and other examples may also provide updated user parameters to a safety module based upon received settings changes. The system monitor of this and other examples may include a subsystem module in communication with one or more subsystems. The subsystem module may be adapted and/or otherwise configured to periodically scan subsystem data from the one or more subsystems and provide actual user parameters to the safety module from the scanned subsystem data. In this and other examples, the safety module may be adapted and/or otherwise configured to compare the updated user parameters to the actual user parameters, and may correct the actual user parameters to match the updated user parameters.

Alternatively or additionally to any of the examples disclosed herein, the safety module may trigger one or more of an alarm and an alert if the actual parameters cannot match the updated user parameters.

Alternatively or additionally to any of the examples disclosed herein, the safety module may be adapted and/or otherwise configured to perform one or more cyclic redundancy checks (CRC) upon the profile module.

Alternatively or additionally to any of the examples disclosed herein, the one or more user parameters may be one or more of: pump settings, motor settings, flow settings, user settings, pressure settings, connection settings, physiological settings, device settings, treatment settings, and procedural settings.

Alternatively or additionally to any of the examples disclosed herein, computer-executable instructions for correcting a user profile and/or role-change discrepancy in a heart-lung machine (HLM) are provided by the disclosure herein. An example provision of computer-executable instructions may include a safety monitor configured to monitor one or more subsystem parameters defined in a profile and/or role change of a user of a heart-lung machine. The safety monitor of this and other examples may be further adapted and/or otherwise configured to: compare the one or more subsystem parameters to one or more previous subsystem parameters defined in a previous profile and/or role change of the user; detect one or discrepancies in the one or more subsystem parameters with respect to the one or more previous subsystem parameters; correct the one or more subsystem parameters having a detected discrepancy to a correct value.

Alternatively or additionally to any of the examples disclosed herein, the safety monitor of this and other examples may trigger one or more of an alarm and an alert if the one or more discrepancies are unable to be corrected to a correct value.

Alternatively or additionally to any of the examples disclosed herein, the safety monitor performs one or more cyclic redundancy checks (CRC) to compare the one or more subsystem parameters to the one or more previous subsystem parameters.

Alternatively or additionally to any of the examples disclosed herein, the one or more subsystem parameters may include one or more of: pump settings, motor settings, flow settings, user settings, pressure settings, connection settings, physiological settings, device settings, treatment settings, and procedural settings.

Alternatively or additionally to any of the examples disclosed herein, the one or more discrepancies may include one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, and a mismatch in procedural settings.

Alternatively or additionally to any of the examples disclosed herein, computer-implemented methods for automatically correcting a user profile and/or role-change discrepancy in a heart-lung machine (HLM) are detailed throughout the present disclosure herein. The methods of this and other examples may include: monitoring one or more subsystem parameters defined in a profile and/or role change of a user of a heart-lung machine; comparing the one or more subsystem parameters to one or more previous subsystem parameters defined in a previous profile and/or role change of the user; detecting one or more discrepancies in the one or more subsystem parameters with respect to the one or more previous subsystem parameters; correcting the one or more discrepancies in the one or more subsystem parameters such that the one or more subsystem parameters are configured accurately.

Alternatively or additionally to any of the examples disclosed herein, methods may include periodically checking the one or more subsystem parameters and confirming that the one or more subsystem parameters are configured accurately and/or present (i.e., available and/or identifiable).

Alternatively or additionally to any of the examples disclosed herein, the safety monitor system may be adapted and/or otherwise configured to alert the user with an alarm if the one or more discrepancies cannot be corrected.

Alternatively or additionally to any of the examples disclosed herein, the one or more subsystem parameters may be one or more of: pump settings, motor settings, flow settings, user settings, pressure settings, connection settings, physiological settings, device settings, treatment settings, and procedural settings.

Alternatively or additionally to any of the examples disclosed herein, the one or more discrepancies may be one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, and a mismatch in procedural settings.

While multiple examples and embodiments are disclosed, still other examples and embodiments of the present disclosure will become apparent to those skilled in the art from the following detailed description, which shows and describes illustrative embodiments and examples of the present disclosure. Accordingly, the drawings and detailed description are to be regarded as illustrative in nature and not restrictive.

### BRIEF DESCRIPTION OF THE SEVERAL VIEWS OF THE DRAWINGS

The drawings illustrate the design and utility of preferred embodiments of the present disclosure. It should be noted that the figures are not drawn to scale and that elements of similar structures or functions are represented by like reference numerals throughout the figures. In order to better appreciate how the above-recited and other advantages and objects of the present disclosure are obtained, a more particular description of the present disclosure briefly described above will be rendered by reference to specific embodiments thereof, which are illustrated in the accompanying drawings. Understanding that these drawings depict only typical embodiments of the disclosure and are not therefore to be considered limiting of its scope, the present disclosure will be described and explained with additional specificity and detail through the use of the accompanying drawings, in which:
FIG. 1A is a front perspective view of an illustrative heart-lung machine (HLM), in accordance with embodiments of the subject matter disclosed herein.
FIG. 1B is a side perspective view of the illustrative HLM depicted in FIG. 1A, in accordance with embodiments of the subject matter disclosed herein.
FIG. 1C is a partial perspective view of the base of the trolley depicted in FIGS. 1A and 1B, in accordance with embodiments of the subject matter disclosed herein.
FIG. 2 is a block diagram depicting an illustrative operating environment of an HLM, in accordance with embodiments of the subject matter disclosed herein.
FIG. 3 is an example diagram depicting the interconnectedness of various system monitor features.

While the disclosure is amenable to various modifications and alternative forms, specifics thereof have been shown by way of example in the drawings and will be described in detail. It should be understood, however, that the intention is not to limit the disclosure to the particular examples described. On the contrary, the intention is to cover all modifications, equivalents, and alternatives falling within the spirit and scope of the disclosure.

### DETAILED DESCRIPTION

The independent system monitor (i.e., system monitor) and related components and/or devices of the present disclosure ensure the correct configuration of the HLM system automatically, reducing the risk of human error and enhancing patient safety. Certain systems often suffer from unrecognized loss or change of user profile data (i.e., profile data) during procedures, leading to user complaints. The independent system monitor and related components and/or devices of the present disclosure may continuously monitor and correct profile data mismatches, ensuring accurate system configuration throughout a procedure. The use of one or more cyclic redundancy checks (CRC) for reviewing data integrity provides a robust method for verifying profile completeness, not commonly found in existing HLM systems. This innovation significantly improves reliability and automation in life-supporting medical devices, addressing an unmet need in the art.

The HLM system 100 can be used for many different types of medical procedures. For example, the medical procedures for which the HLM system 100 can be used include, but are not limited to: coronary artery bypass grafts, heart valve repairs, heart valve replacements, heart transplants, lung transplants, ablation procedures, repair of septal defects, repair of congenital heart defects, repair of aneurysms, pulmonary endarterectomy, pulmonary thrombectomy, and the like.

The HLM system 100 is typically set up and operated by a specially-trained clinician called a perfusionist. Perfusionists form part of the wider cardiovascular surgical team that includes cardiac surgeons, anesthesiologists, and nurses. During medical procedures using the HLM system 100, the perfusionist is tasked with many responsibilities, not the least of which is ensuring that the patient is kept alive and healthy by operating the HLM system 100 in a manner that maintains blood flow to the patient's tissues, and which regulates levels of oxygen and carbon dioxide in the blood of the patient. Other responsibilities of the perfusionist include, but are not limited to, administering blood products, administering anesthetic agents or drugs, measuring selected laboratory values (such as blood cell count), monitoring circulation, monitoring blood gases, surveilling anticoagulation, induction of hypothermia, and hemodilution. The responsibilities of the perfusionist are diverse, dynamic, and critically important to achieving successful outcomes of procedures performed on the patient using the HLM system 100.

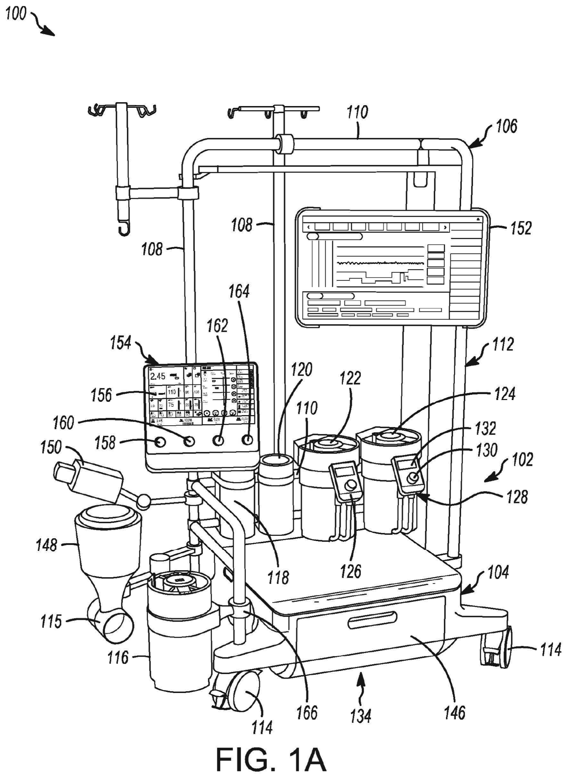

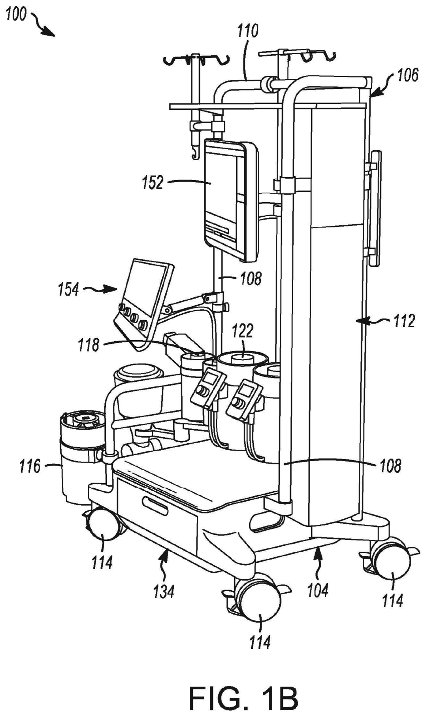

FIG. 1A is a front perspective view of an illustrative heart-lung machine (HLM) 100, in accordance with embodiments of the subject matter disclosed herein; and FIG. 1B is a side perspective view of the illustrative HLM 100 depicted in FIG. 1A, in accordance with embodiments of the subject matter disclosed herein. As shown, the HLM 100 includes a trolley 102 having a base 104 that includes an internal cavity (not shown) for housing any number of different controls, electrical circuits, hydraulic circuits, a battery discharger, and/or the like. For example, in embodiments, a peripheral processing unit may be disposed within the base 104. A mast assembly 106 is coupled to the base 104 and extends upwards from the base 104. The mast assembly 106 may include any number of different mast components, including vertical poles 108, horizontal rails 110, and/or the like. In embodiments, the trolley 102 includes an enclosure 112 that is configured to facilitate cable management, provide A/C outlets, include a power switch for the HLM 100, include an extension box, and/or the like. As shown, the trolley 102 also may include wheels 114 coupled to the base 104.

As shown, the HLM 100 also may include a number of different types of components such as an oxygenator 115 (which may actually be considered to be an element of an extracorporeal circuit used with the HLM, but may be referred to herein as being a component of the HLM due to being connected to the trolley 102); pumps 116, 118, 120, 122, 124; and/or the like. In embodiments, one or more of the components 115, 116, 118, 120, 122, and 124 (and/or others) may be coupled to any number of different portions of the mast assembly 106, and may include, for example, an exposed actuator control unit (ACU). For example, as shown, pumps 122 and 124 may each include an exposed ACU 126 and 128, operably connected thereto, respectively. As shown, an ACU 128 may include a control knob 130 configured to receive user input (e.g., manipulation of the knob 130) for controlling operation of the pump 124, and an information display device 132 configured to present information associated with the pump such as, for example, one or more parameters (e.g., measured device parameters such as, for instance, flow, rpm, etc.). According to embodiments, an ACU may be configured to facilitate control of a pump, a motorized clamp, a motorized occluder, an infusion device, and/or any number of other types of devices that may be associated with an HLM.

Traditionally, HLMs have utilized roller pumps that are each integrated into a modular console component. The modular console components are stacked next to one another on the base of an HLM to provide an array of pumps. The modular console component also houses an ACU having an interface for controlling the corresponding integrated roller pump. One advantage of having the ACU interface provided at the modular console component is that, during an emergency situation, the perfusionist can easily determine the ACU that corresponds to a particular roller pump. More recently, mast mounted roller pumps (without the modular console component housing) have been utilized in HLMs. Mast mounted pumps provide more flexibility in the configuration of the HLM; however, if the ACUs for the mast mounted pumps are located remotely, or detached from, the mast mounted pumps, it's potentially more difficult for the perfusionist (i.e., the user) to identify the ACU that controls a particular pump.

Embodiments of the present disclosure include mast mounted roller pumps, such as pumps 122 and 124 of FIG. 1A, having corresponding ACUs, such as ACUs 126 and 128, respectively. The ACUs 126 and 128 are attached, connected, or otherwise operatively coupled to the corresponding mast mounted roller pumps 122 and 124. The connectedness, or close proximity of the ACU to the mast mounted roller pump allows the user to precisely determine the ACU that controls a particular mast mounted roller pump in a high pressure, or emergency situation, including a situation where the control display device 156 is not functioning properly or is disabled. Thus, the ability to mount the pumps 122 and 124 to the mast assembly 106 allows a much wider range of configurations to meet the user's particular needs.

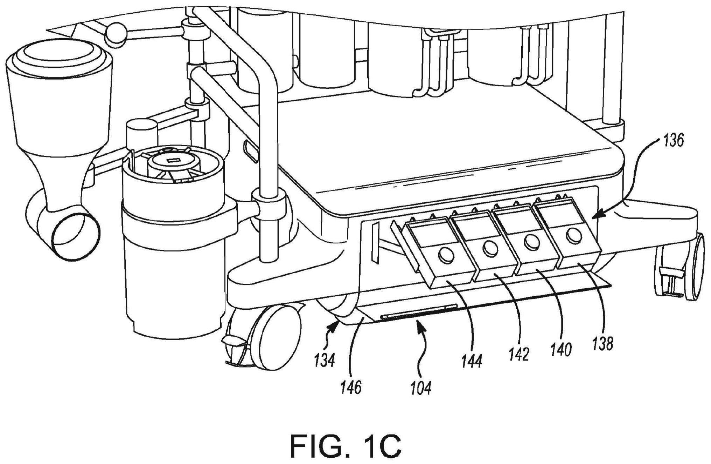

FIG. 1C is a partial perspective view of the base 104 of the trolley 102 depicted in FIGS. 1A and 1B, in accordance with embodiments of the subject matter disclosed herein. As shown in FIG. 1C, the base 104 of the trolley 102 may include a lower housing 134 having an enclosure 136 configured to house one or more ACUs 138, 140, 142, and 144. In embodiments, any number of ACUs may be disposed in the enclosure 136. For example, in embodiments, all of the ACUs for actuators associated with the HLM 100 may be disposed at least partly in the enclosure 136. In embodiments, one or more ACUs may be exposed by being disposed directly on or near the corresponding actuators. According to embodiments, the enclosure 136 may be configured to be closed to protect the ACUs disposed therein, or opened to reveal the ACUs. For example, the lower housing 134 may include a drawer, cabinet, and/or the like. As shown, in embodiments, the lower housing 134 may include a door 146 configured to be opened and closed to selectively expose or conceal the enclosure 136. Each of the ACUs 138, 140, 142, and 144 may be operably connected to a corresponding one of the components 116, 118, 120, 122 (e.g., in cases in which the pump 122 does not include an exposed ACU 126), or 124 (e.g., in cases in which the pump 124 does not include an exposed ACU 128) and/or other actuators.

As is further shown in FIGS. 1A and 1B, the HLM 100 may include any number of other components such as, for example, a venous reservoir 148 (which may be, for example, a component of an extracorporeal circuit that may be used in combination with the HLM 100), an electronic venous occluder (EVO) 150, a peripheral display device 152, a control assembly 154 (which may be interchangeably referred to as "the cockpit"), any number of various types of sensors, and/or the like. According to embodiments, any number of the components discussed herein, others not discussed herein, or aspects of the components (e.g., sensors and/or actuators associated with components) may be operably connected to the peripheral processing unit (not shown), which may be configured to receive parameter data from any one or more of the components, process parameter data, receive control signals from any one or more input devices (e.g., ACU control knobs 130, etc.), provide control signals to any one or more of the components, and/or the like.

In embodiments, the peripheral display device 152 may be operably connected to the peripheral processing unit and configured to present a set of parameter data received from the peripheral processing unit. In embodiments, the peripheral display device 152 may be, include, or be included within a data recording and/or management system. That is, for example, the peripheral display device 152 may include, or be otherwise associated with, a processing unit separate from that of the HLM, and/or may be configured to record and/or display any number of different operative HLM parameters. In some implementations, for example, the peripheral display device 152 may be configured to obtain and record all of the operative HLM parameter values and/or patient parameters provided by any number of additional monitoring devices. The peripheral display device 152 may be configured to present, graphically, representations of any number of the obtained parameter values, changes in parameter values over time, derived parameter values (e.g., values derived from parameter values), and/or the like.

During an operation, the primary focus of a user of the HLM 100 generally is the oxygenator 115 and the venous reservoir 148. Accordingly, embodiments of the subject matter disclosed herein provide a control assembly 154 near those two components 115 and 148 so that the user can access control devices and view displayed parameters without having to move away from, or be distracted from, the oxygenator 115 and venous reservoir 148. According to embodiments, the control assembly 154 may include a control display device 156 and a number of input control devices 158, 160, 162, and 164. In embodiments, the control assembly 154 may include any number of input control devices (e.g., 1, 2, 3, 4, 5, 6, etc.) and the number of input control devices may be less than or equal to the number of ACUs in the enclosure 136. The control display device 156 may be configured to present a subset of the parameters presented by the peripheral display device 152 and/or the peripheral display device 152 may be configured to present a subset of the subset of parameters presented by the control display device 156. A different subset of the set of parameter data may be displayed by the peripheral display device 152. In embodiments, the peripheral display device 152 may be configured to display real-time waveform traces, while the control display device 156 may be configured to display numerical representations of the same and/or different parameters.

That is, for example, regardless of what is displayed on the peripheral display device 152, the control display device 156 may be configured to display a specified subset of parameter data that is particularly useful and/or important with respect to a procedure being performed. That specified subset of parameter data may be predetermined, based on the type of procedure; dynamically presented, based on a status of the patient and/or device; and/or the like. In embodiments, all of the information configured to be presented on the control display device 156 may be presented simultaneously-that is, without having tabs for accessing screens showing additional information, without requiring menus for accessing screens showing additional information during a procedure, and/or the like. In embodiments, the control display device 156 may include selectable representations presented onscreen that can be used to configure the display such as, for example, by enabling a user to select a display mode corresponding to a particular HLM component (e.g., a centrifugal pump, a roller pump, etc.), to select a particular display module (e.g., a pre-configured set of data fields in a particular arrangement), and/or the like.

According to embodiments, the peripheral display device 152 and/or the control display device 156 may include an input mechanism configured to enable user interaction with one or more features displayed on the display device 152 and/or 156. That is, for example, the peripheral display device 152 and/or the control display device 156 may be, or include, a touchscreen device configured to receive user input. In embodiments, the peripheral display device 152 and/or the control display device 156 may include an input device connected thereto such as, for example, a mouse, a trackpad, a joystick, a brain-computer interface (BCI), and/or the like.

According to embodiments, for example, additional data from devices external to the HLM (e.g., blood gas monitors, electrocardiographs, ventilators, patient monitors, etc.) may be displayed on the peripheral display device 152. As indicated above, the peripheral display device 152 may be controlled by a peripheral processing unit that is separate from the central system unit of the HLM. The peripheral processing unit associated with the peripheral display device 152 may be configured to obtain parameter values (e.g., from the central system unit, sensors, actuators, external devices, etc.) and may be configured to collect the data in a database. The peripheral processing unit may be communicatively coupled to the peripheral display device 152, HLM components, and/or external devices. In embodiments, while the peripheral processing unit may be configured to receive data from the central system unit, an interface unit or any other communication port embedded in the HLM, the peripheral processing unit may be configured so as to not send any data to the central system unit, to the interface unit or other communication ports of the HLM. In other embodiments, the peripheral processing unit, the central system unit, the interface unit or any other communication port of the HLM may be configured to exchange data with one another and/or other devices. According to embodiments, a user may select which data is to be stored by which processing or system unit.

The peripheral processing unit associated with the peripheral display device may be configured to allow user interaction therewith, generate reports based on the obtained data, generate printable documents corresponding to a medical procedure, interact with a printer to cause the printer to print such reports, and/or the like. In embodiments, the peripheral processing unit may be configured to generate, and cause the peripheral display device to present, graphs (e.g., trend charts, curves, etc.) and/or other visual representations of any number of various aspects of data received from HLM components and/or external devices. In embodiments the peripheral display device may be configurable such that a user can select certain types of data and/or representations thereof to display, the manner in which it is displayed, and/or the like. In contrast, for example, the control display device 156 may include only limited configurability, if at all. In this manner, the control display device 156 can be relied upon to present representations of data relevant to the HLM's current use. According to other embodiments, the control display device 156 may have any amount of configurability.

In embodiments, each of the input control devices 158, 160, 162, and 164 may be operably connected to one of the actuators and may be configured to receive user input for controlling an operation of the actuator. According to embodiments, the input control devices 158, 160, 162, and 164 may be operably connected to the respective ACUs 138, 140, 142, and 144, in which case, the input control devices 158, 160, 162, and 164 act in parallel to the ACUs, but do not have priority over them in controlling the actuators. In embodiments, the input control devices are directly connected to the respective ACUs, and the ACUs are connected to the respective actuators, such that an actuator can be controlled by an input control device only through an ACU or directly by an ACU. Therefore, the ACU has prevalence over the input control device in controlling the actuator.

As is further shown in FIG. 1A, the HLM 100 may include a connection assembly 166 configured to facilitate connecting at least one HLM component to the HLM base 104. The connection assembly 166 may include any number of different HLM component connectors configured to facilitate removably connecting an HLM component to the HLM base 104. Each HLM component connector may include any number of connection elements configured to facilitate operatively connecting an HLM component to other components of the HLM. In embodiments, the connection elements may include fluid connection elements, energy connection elements, and/or data connection elements. In embodiments, the control assembly 154 may be configured to facilitate configuring the connection assembly 166. According to embodiments, configuring the connection assembly 166 may include, for example, providing input to a control unit via the control assembly 154 that assigns a particular type of HLM component to the connection assembly 166 and/or a connector thereof, assigns a particular HLM component to the connection assembly 166 and/or a connector thereof, causes the control assembly to display a representation of the connector and/or HLM component, causes the control assembly to include data received via the connector to be displayed in a user interface, and/or the like.

According to embodiments, any one or more of the components of the illustrative HLM 100 may be implemented on one or more computing devices. A computing device may include any type of computing device suitable for implementing aspects of embodiments of the disclosed subject matter. Examples of computing devices include specialized computing devices or general-purpose computing devices such "control units," "control assemblies," "workstations," "servers," "hand-held devices," "heart-lung machines," "controllers," and the like, all of which are contemplated within the scope of FIG. 1, with reference to various components of the HLM 100.

In embodiments, a computing device includes a bus that, directly and/or indirectly, couples the following devices: a processing unit, a memory, an input/output (I/O) port, an I/O component, and a power supply. Any number of additional components, different components, and/or combinations of components may also be included in the computing device. The I/O component may include a presentation component configured to present information to a user such as, for example, a display device, a speaker, a printing device, and/or the like, and/or an input component such as, for example, a microphone, a joystick, a satellite dish, a scanner, a printer, a wireless device, a keyboard, a pen, a voice input device, a touch input device, a touch-screen device, an interactive display device, a mouse, and/or the like.

The bus represents what may be one or more busses (such as, for example, an address bus, data bus, or combination thereof). Similarly, in embodiments, the computing device may include a number of processing units, a number of memory components, a number of I/O ports, a number of I/O components, and/or a number of power supplies. Additionally any number of these components, or combinations thereof, may be distributed and/or duplicated across a number of computing devices.

In embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

The illustrative HLM 100 shown in FIGS. 1A-1C is not intended to suggest any limitation as to the scope of use or functionality of embodiments of the present disclosure. The illustrative HLM 100 also should not be interpreted as having any dependency or requirement related to any single component or combination of components illustrated therein. Additionally, various components depicted in FIGS. 1A-1C may be, in embodiments, integrated with various ones of the other components depicted therein (and/or components not illustrated), all of which are considered to be within the ambit of the present disclosure.

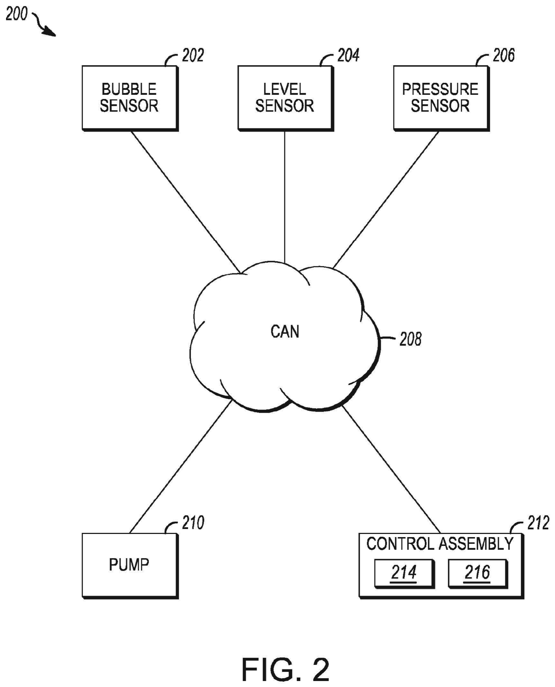

FIG. 2 is a block diagram depicting an illustrative operating environment 200, in accordance with embodiments of the subject matter disclosed herein. According to embodiments, the illustrative operating environment 200 may be implemented on an HLM or aspects thereof such as, for example, the HLM 100 depicted in FIGS. 1A-1C. As shown, the operating environment 200 includes a number of sensors: a bubble sensor 202, a level sensor 204, and a pressure sensor 206. Each of the sensors 202, 204, and 206 may be communicatively coupled to a control area network (CAN) 208. Any number of other types of sensors may be included such as, for example, flow sensors, temperature sensors, blood gas sensors, and/or the like.

A system pump 210 is communicatively coupled to the CAN 208. The system pump 210 may be any kind of fluid pump configured to facilitate performing a perfusion task such as, for example, a roller pump or a centrifugal pump. A control assembly 212 is communicatively coupled to the CAN 208. In embodiments, the control assembly 212 may be, include, be included in, or be similar to the control assembly 154 depicted in FIGS. 1A and 1B. In embodiments, the control assembly 212 may include a processing unit 214 and a memory 216. The processing unit 214 may, for example, be configured to control a control display device by, for example, providing the parameters to display, providing the defining of a layout of display elements, process user input, and/or the like.

In embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

The illustrative operating environment 200 shown in FIG. 2 is not intended to suggest any limitation as to the scope of use or functionality of embodiments of the present disclosure. The illustrative operating environment 200 also should not be interpreted as having any dependency or requirement related to any single component or combination of components illustrated therein. Additionally, various components depicted in FIG. 2 may be, in embodiments, integrated with various ones of the other components depicted therein (and/or components not illustrated), all of which are considered to be within the ambit of the present disclosure.

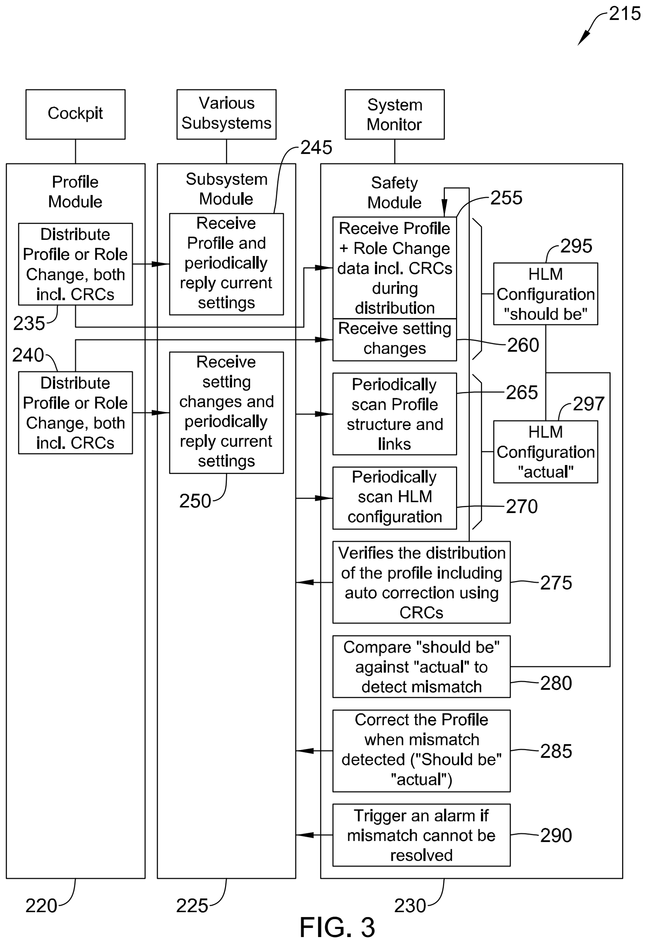

According to embodiments, the instructions may be configured to cause the processing unit 214, upon being executed by the processing unit 214, to facilitate presenting a safety check on a display or other like user interface (such as user interface UI). Alternatively or additionally, a safety check may be performed by a system monitor 215, see FIG. 3, disclosed herein. A system monitor 215 of this and other examples may include a profile module 220 configured and/or otherwise adapted to distribute and/or otherwise provide user parameters and/or profile and/or role changes 235 and send settings changes 240 from a remote interface (e.g., from the central system unit, sensors, actuators, user displays, external devices, etc.) to a subsystem module 225. The subsystem module 225 of this and other examples may receive profile settings 245 and periodically reply to the profile module 220 with current settings 245. Alternatively or additionally, the subsystem module 225 of this and other examples may receive setting changes and periodically reply to the profile module 220 with current settings 250 reflecting the settings changes 240 sent by the profile module 220 to the subsystem module 225. In other words, the subsystem module 225 may be adapted and/or otherwise configured to receive profile (i.e., user profile) settings, and periodically reply to the profile module 220 with the current settings 245 (i.e., the operational and/or procedural and/or user settings currently applied to the various subsystems) of the various subsystems, including but not limited to the various subsystems disclosed herein.

The subsystem module 225 of the system monitor 215 may be in communication with one or more subsystems and may be in direct and/or indirect communication with one or more of the profile module 220 and safety module 230. In this and other examples, the safety module 230 may be configured and/or otherwise adapted to periodically scan subsystem data (265, 270), and may periodically scan subsystem data for elements and/or data features regarding user profile structure and links 265. The safety module 230 of this and other examples may periodically scan the HLM configuration 270 of a heart-lung machine and/or other like device including but not limited to related, associated and/or peripheral components and/or devices. The safety module 230 may utilize the scanned subsystem data (265, 270) from the various subsystems disclosed and provide actual user parameters 297 to the other communicative elements and/or features of the heart-lung machine and/or other like devices. It can be appreciated that the scanned subsystem data includes but is not limited to the data provided to and/or received from the various subsystems as disclosed herein.

The system monitor 215 of this and other examples may be configured and/or otherwise adapted to compare the updated user parameters 295 (i.e., the preferred, optimal and/or recommended parameter values of which should govern the contemporaneous operation of the various subsystems and/or the like) to the actual user parameters 297 (i.e., the actual parameter values governing contemporaneous operation of the various subsystems and/or the like), and correct the actual user parameters 297 to match the updated user parameters 295. Alternatively or additionally, the system monitor 215 may receive user profile and/or role change data (including cyclic redundancy checks) during distribution of user profile and/or user role change data 235 from the profile module 220, and may also receive the sent settings changes 240 from the profile module 220, resulting in received settings changes 260 as shown in FIG. 3. The safety module 230 may further utilize the received user profile and/or role change data 255 and the received settings changes 260 to calculate the updated user profile settings and/or parameters 295. The system monitor 215 and/or the safety module 230 may alternatively or additionally periodically scan the user profile and/or user role change data 265, and also may periodically scan the configuration(s) of the HLM and/or other like and/or related components 270 to calculate the actual user profile parameters and/or actual user parameters and/or actual parameters 270.

Alternatively or additionally, the system monitor 215 of any of the examples disclosed herein may trigger one or more of an alarm 290 and/or an alert if the actual user parameters 297 cannot match the updated user parameters 295. Alarms 290 and/or alerts may include, but are not limited to: visual alarms, audio alarms, verbal alarms, AI-generated alarms, electronic alarms, mechanical alarms, intermittent alarms, timed alarms, repeating alarms, programmed alarms, visual alerts, audio alerts, verbal alerts, AI-generated alerts, electronic alerts, mechanical alerts, intermittent alerts, timed alerts, repeating alerts, programmed alerts, and/or any of the like, and/or any combination and/or permutation of the aforementioned alarms and/or alerts.

Alternatively or additionally, the system monitor 215 of any of the examples disclosed herein may be configured and/or otherwise adapted to perform one or more cyclic redundancy checks (CRC) upon the profile module. Cyclic redundancy checks (CRC) may include but may not be limited to any error-detecting code used to detect accidental changes to data. Alternatively or additionally, cyclic redundancy checks may include but are not limited to binary cyclic redundancy checks, non-binary cyclic redundancy checks, n-bit binary cyclic redundancy checks, cyclic redundancy checks utilizing checksums, CRC-8, CRC-16, CRC-32, hash functions, checksums, and/or any of the like, and/or any combination and/or permutation of the aforementioned.

Alternatively or additionally, the system monitor 215 of any of the examples disclosed herein, the one or more user parameters may include, but are not limited to one or more of: pump settings, motor settings, flow settings, user settings, pressure settings, connection settings, physiological settings, device settings, treatment settings, and procedural settings. In other words, any applicable setting values (i.e., pump speed, motor speed, blood flow rate, patient blood pressure, lung oxygenation rate, etc.) may be applicable as settings applied both to the user of the devices disclosed herein, and to the devices disclosed herein.

Alternatively or additionally to any of the examples disclosed herein, computer-executable instructions for correcting a user profile and/or role-change discrepancy in a heart-lung machine (HLM) may be provided. The computer-executable instructions of this and other examples may include a safety monitor 215 configured and/or otherwise adapted to monitor one or more subsystem parameters defined in a profile and/or role change 235 of a user and/or practitioner of a heart-lung machine. In other words, the safety monitor 215 of this and other examples may monitor one or more subsystem parameters including but not limited to the subsystem parameters disclosed herein.

The system monitor 215 of this and other examples may be configured to and/or otherwise adapted to compare the one or more subsystem parameters to one or more previous subsystem parameters defined in a previous profile and/or role change of the user. In other words, the system monitor 215 of this and other examples may compare the one or more actual user parameters 297 and/or actual subsystem parameters to the one or more updated user parameters 295 and/or updated subsystem parameters. It can be appreciated that the one or more actual user parameters 297 and/or actual subsystem parameters may be any parameter, setting and/or value disclosed herein. It can be further appreciated that the one or more updated user parameters 295 and/or updated subsystem parameters may be any parameter, setting and/or value disclosed herein.

The system monitor 215 of this and other examples may be configured to and/or otherwise adapted to detect one or more discrepancies and/or mismatches by comparing (see block 280 in FIG. 3) the updated user parameters and/or user profile settings 295 to the actual user parameters and/or user profile settings 297, and may correct (see block 285 in FIG. 3) the one or more subsystem parameters having a detected discrepancy to a correct value. In other words, the system monitor 215, and in some embodiments the safety module 230, may be configured to and/or otherwise adapted to detect one or more mismatches and/or discrepancies between the updated user parameters and/or user profile settings 295 and the actual user parameters and/or user profile settings 297 that are contemporaneously being utilized for operation of the HLM and/or other like devices and/or related devices and/or components described herein.

In this and other examples, the safety monitor may trigger one or more of an alarm 290 and an alert if the one or more discrepancies are unable to be corrected to a correct value. In other words, a user and/or practitioner and/or anyone associated and/or within a near distance to the HLM and/or associated devices disclosed herein may be alerted by an alarm 290 and/or an alert emanating and/or otherwise issuing from any component and/or device described herein. Alarms 290 and/or alerts may include, but are not limited to: visual alarms, audio alarms, verbal alarms, AI-generated alarms, electronic alarms, mechanical alarms, intermittent alarms, timed alarms, repeating alarms, programmed alarms, visual alerts, audio alerts, verbal alerts, AI-generated alerts, electronic alerts, mechanical alerts, intermittent alerts, timed alerts, repeating alerts, programmed alerts, and/or any of the like, and/or any combination and/or permutation of the aforementioned alarms and/or alerts.

Alternatively or additionally, the system monitor of this and other examples may perform one or more cyclic redundancy checks (CRC) to compare the one or more subsystem parameters to the one or more previous subsystem parameters. Cyclic redundancy checks (CRC) may include but may not be limited to any error-detecting code used to detect accidental changes to data. Alternatively or additionally, cyclic redundancy checks may include but are not limited to binary cyclic redundancy checks, non-binary cyclic redundancy checks, n-bit binary cyclic redundancy checks, cyclic redundancy checks utilizing checksums, CRC-8, CRC-16, CRC-32, and/or any of the like, and/or any combination and/or permutation of the aforementioned. It can be appreciated that alternatives to cyclic redundancy checks may be applied to any of the components and/or features and/or methods disclosed herein, including but not limited to the utilization of checksum and/or hash functions which may be used and/or utilized for the purposes applicable to any of the cyclic redundancy check functionality disclosed herein.

Alternatively or additionally, the subsystem parameters of this and other examples may include, but are not limited to, one or more of: pump settings, motor settings, flow settings, user settings, pressure settings, connection settings, physiological settings, device settings, treatment settings, procedural settings, any combination and/or permutation of the aforementioned, and/or any settings disclosed herein.

Alternatively or additionally, the one or more discrepancies of any of the examples disclosed herein may include but are not limited to one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, a mismatch in procedural settings, or any mismatch between any two or more settings disclosed herein.

The heart-lung machine (HLM) of this and other examples may include various subsystems and their configurations, and their configurations may be referred to as profiles. Various subsystems may include, but are not limited to: pumps, sensors, motors, actuators, user interfaces, I/O devices, rotating devices, mechanical devices, fluid-driven devices, electronic devices, electrochemical devices, electromechanical devices, computational devices, handheld devices, ambulatory devices, modular devices, portable devices, disposable devices, and/or the like and/or any combination and/or permutation of the aforementioned. Pumps may include but are not limited to: roller pumps, centrifugal pumps, blender pumps, vacuum pumps, fluid pumps, pneumatic pumps, mechanical pumps, electric pumps, electromechanical pumps, piezoelectric pumps, and/or any combination of the aforementioned and/or the like.

Example actuators may include but are not limited to: occluders, gas blenders, vacuum controllers, vacuum actuators, mechanical actuators, pneumatic actuators, electronic actuators, piezoelectric actuators, chemical actuators, fluid actuators, electromechanical actuators, electrochemical actuators, and/or any combination and/or permutation of the aforementioned, and/or the like.

The profile module 220 also may interchangeably be referred to as a cockpit, and may allow users and/or practitioners of the devices disclosed herein to select a profile for different types of external perfusion cases. The profile module 220 (i.e., cockpit) may also allow users to set system preferences, alarm limits for sensors, general settings, physiologic settings, and/or any settings and/or user parameters and/or values disclosed herein, including but not limited to any combination and/or permutation of the aforementioned and/or the like. Alternatively or additionally, the profile module 220 (i.e., cockpit)may monitor the external perfusion process, in other words, the cockpit 220 and/or profile module 220 may be provided in the form of a user interface in non-limiting examples. The cockpit 220 and/or profile module 220 of this and other examples may display all values and settings, notifications, and alarms and/or alerts issuing from and/or associated with the heart-lung machine 100 and/or other like device(s).

Alternatively or additionally, one or more checksum or hash functions described herein could be utilized instead of one or more cyclic redundancy checks (CRC) for verifying the completeness and integrity of the profiles, such as MD5 and/or SHA-1.

Any of the examples disclosed herein, including any and all components, functionality and elements may be incorporated by methods of the present disclosure. Methods disclosed herein may include computer-implemented methods utilizing and/or otherwise incorporating any and/or all components, functionality and elements of the present disclosure. In some examples, a computer-implemented method for automatically correcting a user profile and/or role-change discrepancy in a heart-lung machine (HLM) may include monitoring one or more subsystem parameters defined in a profile and/or role change 275 of a user of a heart-lung machine (HLM) 100.

Methods of this and other examples may include comparing the one or more subsystem parameters 295 to one or more previous subsystem parameters 297 defined in a previous profile and/or role change 235 of the user. Alternatively or additionally, a computer-implemented method of this and other examples may detect one or more discrepancies 280 in the one or more subsystem parameters 295 with respect to the one or more previous subsystem parameters 297. Methods of this and other examples may include correcting 285 the one or more discrepancies 280 in the one or more subsystem parameters 295 such that the one or more subsystem parameters 295 are configured correctly and/or accurately.

Alternatively or additionally to any of the examples disclosed herein, computer-implemented methods of the present disclosure may include periodically checking (265, 270) the one or more subsystem parameters (295, 297) and confirming that the one or more subsystem parameters (295, 297) are configured accurately and/or present.

Alternatively or additionally to any of the examples disclosed herein, the safety monitor 215 may be configured and/or otherwise adapted to alert the user with an alarm 290 if the one or more discrepancies 280 cannot be corrected.

Alternatively or additionally to any of the examples disclosed herein, the one or more subsystem parameters (295, 297) may be one or more of: pump settings, motor settings, flow settings, user settings, pressure settings, connection settings, physiological settings, device settings, treatment settings, and procedural settings.

Alternatively or additionally to any of the examples disclosed herein, the one or more discrepancies 280 may be one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, and a mismatch in procedural settings.

Other methods may be incorporated within or in addition to any of the examples disclosed herein. Including but not limited to implementing a manual override system such that users of the devices and/or components and/or heart-lung machine and/or other related devices can manually verify and correct mismatches.

It should be noted and can be appreciated that some of the FIGS. are schematic in nature and are not drawn to scale. Certain features are shown larger than their scale and certain features are omitted from some views for ease of illustration.

It should also be noted that, as used in this specification and the appended claims, the singular forms include the plural unless the context clearly dictates otherwise. It should also be noted that the term "or" is generally employed in its sense including "and/or" unless the content clearly dictates otherwise.

The present disclosure has been described with reference to various specific and exemplary embodiments. Those skilled in the art will understand that changes may be made in details, particularly in matters of shape, size, material and arrangement of parts. Accordingly, various modifications and changes may be made to the examples and the embodiments. Additional or fewer components may be used, depending on the condition that is being treated by the electrosurgical ablation device and other related devices and components disclosed herein. It should be understood that many variations and modifications may be made while remaining within the spirit and scope of the present disclosure. The specifications and drawings are, therefore, to be regarded in an illustrative rather than a restrictive sense.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

## Claims

1. A system monitor for a heart-lung machine (HLM), comprising:
a profile module configured to accept user parameters and receive settings changes from a remote interface, and provide updated user parameters to a safety module based upon received settings changes; and
a subsystem module in communication with one or more subsystems, the subsystem module configured to periodically scan subsystem data from the one or more subsystems and provide actual user parameters to the safety module from the scanned subsystem data;
wherein the safety module is configured to compare the updated user parameters to the actual user parameters, and correct the actual user parameters to match the updated user parameters.

2. The system monitor of claim 1, wherein the safety module triggers one or more of an alarm and an alert if the actual user parameters cannot match the updated user parameters.

3. The system monitor of claim 1 or 2, wherein the safety module is configured to perform one or more cyclic redundancy checks (CRC) upon the profile module.

4. The system monitor of any one of the preceding claims, wherein the one or more user parameters are one or more of: pump settings, motor settings, flow settings, user settings, pressure settings, connection settings, physiological settings, device settings, treatment settings, and procedural settings.

5. The system monitor of any one of the preceding claims, wherein the one or more discrepancies are one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, and a mismatch in procedural settings.

6. Computer-executable instructions for correcting a user profile and/or role-change discrepancy in a heart-lung machine (HLM), the computer-executable instructions including:
a safety monitor configured to monitor one or more subsystem parameters defined in a profile and/or role change of a user of a heart-lung machine;
the safety monitor further configured to:
compare the one or more subsystem parameters to one or more previous subsystem parameters defined in a previous profile and/or role change of the user;
detect one or more discrepancies in the one or more subsystem parameters with respect to the one or more previous subsystem parameters; and:
correct the one of more subsystem parameters having a detected discrepancy to a correct value.

7. The computer-executable instructions of claim 6, wherein the safety monitor triggers one or more of an alarm and an alert if the one or more discrepancies are unable to be corrected to a correct value.

8. The computer-executable instructions of claim 6 or 7, wherein the safety monitor performs one or more cyclic redundancy checks (CRC) to compare the one or more subsystem parameters to the one or more previous subsystem parameters.

9. The computer-executable instructions of any one of claims 6 to 8, wherein the one or more subsystem parameters are one or more of: pump settings, motor settings, flow settings, user settings, pressure settings, connection settings, physiological settings, device settings, treatment settings, and procedural settings.

10. The computer-executable instructions of any one of claims 6 to 9, wherein the one or more discrepancies are one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, and a mismatch in procedural settings.

11. A computer-implemented method for automatically correcting a user profile and/or role-change discrepancy in a heart-lung machine (HLM), the method comprising:
monitoring one or more subsystem parameters defined in a profile and/or role change of a user of a heart-lung machine;
comparing the one or more subsystem parameters to one or more previous subsystem parameters defined in a previous profile and/or role change of the user;
detecting one or more discrepancies in the one or more subsystem parameters with respect to the one or more previous subsystem parameters;
correcting the one or more discrepancies in the one or more subsystem parameters such that the one or more subsystem parameters are configured accurately.

12. The method of claim 11, further including:
periodically checking the one or more subsystem parameters and confirming that the one or more subsystem parameters are configured accurately and/or present.

13. The method of claim 11 or 12, wherein the safety monitor system is configured to alert the user with an alarm if the one or more discrepancies cannot be corrected.

14. The method of any one of claims 11 to 13, wherein the one or more subsystem parameters are one or more of: pump settings, motor settings, flow settings, user settings, pressure settings, connection settings, physiological settings, device settings, treatment settings, and procedural settings.

15. The method of any one of claims 11 to 14, wherein the one or more discrepancies are one or more of: a mismatch in pump settings, a mismatch in motor settings, a mismatch in flow settings, a mismatch in user settings, a mismatch in pressure settings, a mismatch in connection settings, a mismatch in physiological settings, a mismatch in device settings, a mismatch in treatment settings, and a mismatch in procedural settings.
